# EUROPEAN PATENT APPLICATION

(11) **EP 1 203 822 A1**
(43) Date of publication of application: **08.05.2002**
(21) Application number: 00203843.8
(22) Date of filing: 02.11.2000
(51) Int. Cl.: C12P 19/04, C08B 37/00, A23L 1/054, A23K 1/16, C12N 1/20

(54) **Extracellular polysaccharide from Gluconacetobacter spp**

(71) Applicant: SOCIETE DES PRODUITS NESTLE S.A., 1800 Vevey (CH)
(72) Inventor: Duboc, Philippe, 1006 Lausanne (CH); Boesch, Cornelia, 8005 Zürich (CH); Cavadini, Christof, 1801 Le Mont-Pelerin (CH); Fischer, Monica, 1073 Savigny (CH); Hammes, Walter P., 70794 Filderstadt (DE); Schueller, Gerd, 70567 Stuttgart (DE); Stingele, Francesca, 1018 Lausanne (CH); Vincent, Sébastien, 1009 Pully (CH)

(57) **Abstract**

The present invention is related to a bacterial extracellular polysaccharide, which has a xanthan-like structure with a non-cellulosic backbone with 4-beta and 6-beta linkages, non or very little acetylation and having at least one sidechain per repeating unit, an isolated *Gluconacetobacter spp.* strain producing such polysaccharide and to their use in various food or pet food products or for the preparation of food or pet food products.

## Description

### Field of the invention

The present invention is related to extracellular polysaccharides, especially xanthan-like, and to their use in various food products or for the preparation of food or pet food products. It also relates to a *Gluconacetobacter spp.* strain producing such polysaccharide.

### Background of the Invention

Microbial extracellular polysaccharides are long-chain, high-molecular-mass polymers that are secreted into the environment by a large variety of different bacteria. Such microbial polymers, especially xanthan, are widely used in the food industries as agents for thickening, gelling, texturising, suspending and encapsulating (Griffin, A.M. et al., (1996b) FEMS Microbiol. Lett. 137: 115-121; Sutherland, I.W., and Tait, M.I. (1992) Bipolymers. In J. Lederberg (ed.), Enzyclopedia of microbiology, Academic Press, Inc., San Diego, Calif.).

Many extracellular polysaccharides secreted by bacteria are regular structures composed of complex chemical repeat units. These repeat units can contain a number of different sugars linked in a variety of ways; the structure can be branched or multibranched.

The capacity to produce cellulose was attributed to strains of *Gluconacetobacter xylinus* (formerly *Acetobacter xylinum* or *Acetobacter xylinus).* In addition, some strains are able to produce the complex acidic extracellular polysaccharide "acetan" which resembles the widely used polysaccharide xanthan and contains glucose, mannose, glucuronic acid and rhamnose at a molar ratio of 4:1:1:1. Acetan consists of a backbone of cellulose, to which a pentasaccharide branch is bound every two glucose residues (figure 2).

Other strains of *Gluconacetobacter xylinus* were shown to produce different polysaccharides: *A. xylinus* B42 secretes both cellulose and acetan (Petroni et al., (1996) Cell Mol Biol., 42(5):759-67), a mutant of *A*. *xylinus* B42, CR1/4, secretes a polysaccharide with a sidechain shorter than acetan (Colquhoun et al., (1995) Carbohydr Res., 269(2):319-31), *G. xylinus* NCI 1005 secretes a β2→6-fructan (levan) when grown on sucrose (Tajima, K. et al., (1997) Macromol. Symp. 120, 19-28), while the same *G. xylinus* NCI 1005 secretes acetan when grown on glucose (Tayama, K. et al, (1986) Agric. Biol. Chem. 50 1271-1278).

Moreover, it is widely known that the functional properties of an extracellular polysaccharide are determined by its primary structure. Thus, it would be of interest to provide novel structures of extracellular polysaccharides, that would present interesting properties for food industries.

### Summary of the Invention

Accordingly, in a first aspect the present invention aims to provide a bacterial extracellular polysaccharide, which has a xanthan-like structure with a non-cellulosic backbone with 4-beta and 6-beta linkages, non or very little acetylation and having at least one sidechain per repeating unit.

In a preferred embodiment the non-cellulosic backbone may have a repeating unit with the following structure: { [→4)-β-D-G1c-(1→4)-β-D-G1c-(1→]ₘ→6)-β-D-G1c-(1→4)-β-D-G1c-(1→ }n, in which *m* is an integer between 1 and 10, and *n* another integer defining the number of repeating units.

The sidechain(s) may be identical or different and can be αL-Rha(1→6)-βD-G1c-(1→6)-αD-Glc-(1→4)- βD-G1cA-(1→2)- αD-Man, βD-G1c-( 1→6)-αD-G1 c-( 1→4)-βD-G1cA-( 1→2)-αD-Man, αD-G1c-(1→4)-βD-G1cA-(1→2)- αD-Man, βD-G1cA-(1→2)- αD-Man, αD-Man, αL-Rha(1→6)-βD-G1c-(1→6)-αD-G1c-(1→4)- βD-G1cA, βD-G1c-(1→6)-αD-G1c-(1→4)- βD-G1cA, αD-G1c-(1→4)- βD-G1cA, βD-G1cA, αL-Rha(1→6)-βD-G1c-(1→6)-αD-G1c, βD-G1c-(1→6)-αD-G1c, αD-G1c, αL-Rha(1→6)-βD-G1c, αL-Rha, βD-G1c, or their derivatives, for example.

The acetylation may be less than 0.8 per site, and preferably less than 0.5 per site. The possible sites on the polysaccharide can be mannose on sidechain or glucose on the backbone chain.

In a preferred embodiment, the polysaccharide has at least two different sidechains per repeating unit, from which at least one is equivalent to the acetan sidechain or mutants of acetan sidechain and one different.

In another embodiment, the polysaccharide has three different sidechains per repeating unit, from which two are equivalent to usual acetan sidechain and one different.

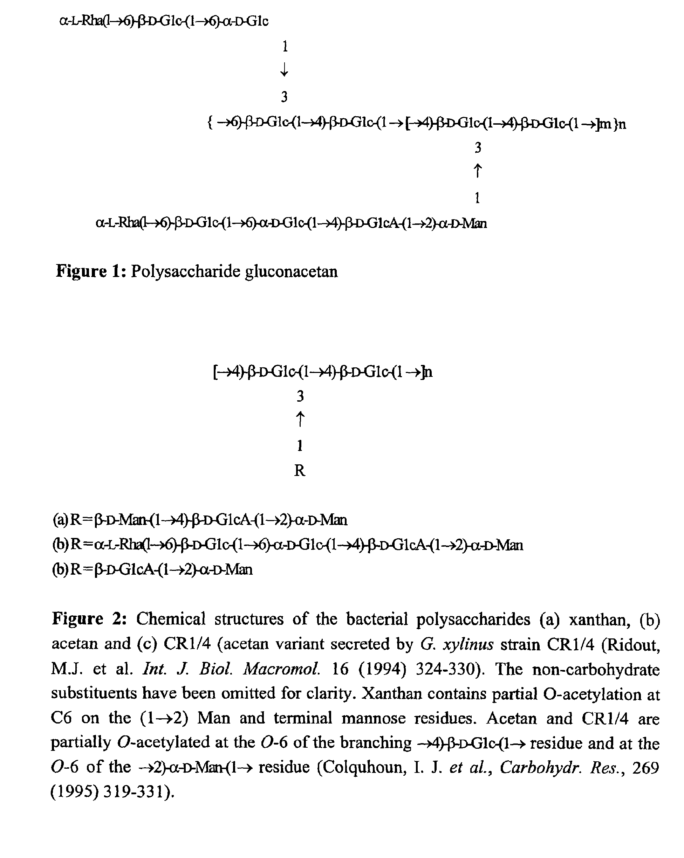

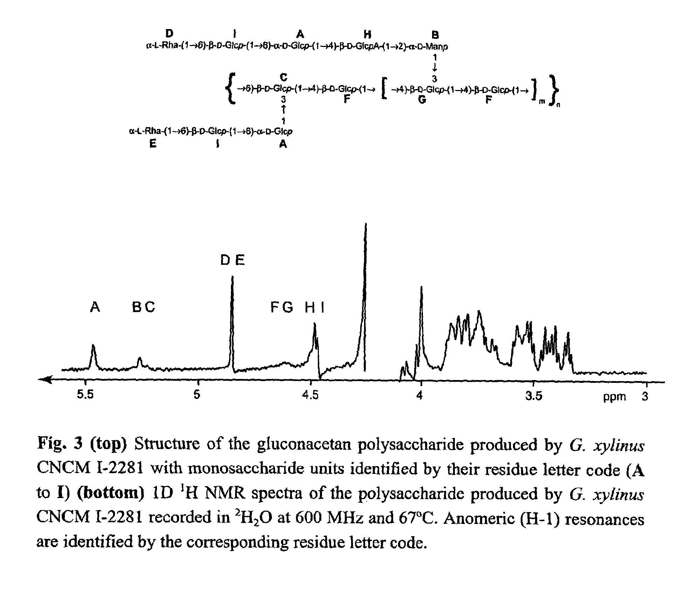

In a most preferred embodiment, the polysaccharide is gluconacetan and has the structure presented in figure 1.

It may be obtained by the strain *Gluconacetobacter* spp. having the deposit number CNCM I-2281.

In another aspect, the present invention aims to provide an isolated and purified *Gluconacetobacter* strain that is characterised by a high production of a polysaccharide as described above and a low or no production of cellulose, which allows its application in various fields, especially the preparation of food or pet food products or its incorporation in food or pet food products.

Preferably, the *Gluconacetobacter* strain is *Gluconacetobacter spp.* having the deposit number CNCM I-2281.

In a further aspect, the invention relates to the use of such polysaccharide and/or said strain in various food or pet food products or for the preparation of various food or pet food products. The polysaccharide according to the invention could also be used as an intermediate product for the isolation of rhamnose that allows increased levels of flavour in various food or pet food products.

The invention also relates to a food or pet food product or composition comprising at least the polysaccharide as described above and/or the bacterial strain as described above.

In a last aspect, the present invention is related to a fermentation process that permits advantageously the control of optimum biomass concentration and polysaccharide concentration by the *Gluconacetobacter* spp. strain according to the invention, wherein said *Gluconacetobacter* spp. strain is maintained under agitation in a growth medium containing salts and two different substrates S1 and S2 as carbon sources, S1 being a source of carbon for the production of biomass and S2 being a source of carbon for the production of polysaccharide.

### Detailed Description of the Invention

Within the following description, the following abbreviations have been used: CCDD, cross-correlated dipole-dipole NMR nuclear magnetic resonance experiment for determining glycosidic linkages; Gal*p*, galactopyranose; Glc*p*A, glucuronic acid pyranose; Glc*p*, glucopyranose; HMBC, heteronuclear multiple-bond correlation; HPLC, high-performance liquid chromatography; PEP-HSQC, preservation of equivalent pathways in heteronuclear single-quantum coherence; NMR, nuclear magnetic resonance; NOESY, nuclear Overhauser effect spectroscopy ; Rha, rhamnose; TOCSY, total correlation spectroscopy; TPPI, time proportional phase increments.

According to a first aspect, a bacterial polysaccharide, which has a xanthan-like structure with a non-cellulosic backbone with 4-beta and 6-beta linkages, non or very little acetylation and having at least one sidechain per repeating unit, is concerned.

The non-cellulosic backbone may have a repeating unit with the following structure: { [→4)-β-D-G1c-(1→4)-β-D-G1c-(1→]ₘ →6)-β-D-G1c-(1→4)-β-D-G1c-(1→ }n, in which *m* is an integer between 1 and 10, but preferably equal to 2, and *n* another integer defining the number of repeating units.

The sidechain(s) may be identical or different and can be : αL-Rha(1→6)-βD-G1c-(1→6)-αD-G1c-(1→4)-βD-G1cA-(1→2)- αD-Man, βD-G1c-( 1 →6)-αD-G1c-( 1 →4)-βD-G1cA-(1 →2)-αD-Man, αD-G1c-(1→4)-βD-G1cA-(1→2)-αD-Man, βD-G1cA-(1→2)- αD-Man, αD-Man, αL-Rha(1→6)-βD-G1c-(1→6)-αD-G1c-(1→4)-βD-G1cA, βD-G1c-(1→6)-αD-G1c-(1→4)- βD-G1cA, αD-G1c-(1→4)- βD-G1cA, βD-G1cA, αL-Rha(1→6)-βD-G1c-(1→6)-αD-G1c, βD-G1c-(1→6)-αD-G1c, αD-G1c, αL-Rha(1→6)-βD-G1c, αL-Rha, βD-G1c, or their derivatives, for example.

The acetylation may be less than 0.8 per site, and preferably less than 0.5 per site. The possible sites on the polysaccharide can be mannose on sidechain or glucose on the backbone chain.

In a preferred embodiment, the polysaccharide has at least two different sidechains per repeating unit, from which at least one is equivalent to the usual acetan sidechain or mutants of acetan sidechain and one different.

In another preferred embodiment, the polysaccharide according to the invention has three sidechains per repeating unit, an unusual property for a bacterial polysaccharide. Two of the sidechains may be equivalent to usual acetan or mutants of acetan sidechain and one different.

In a most preferred embodiment, the polysaccharide is gluconacetan which has the primary structure presented in figure 1. The polysaccharide may be obtained by the strain *Gluconacetobacter* spp. having the deposit number CNCM I-2281.

In contrast to both acetan and xanthan, the backbone residue was found to contain a β1→6 linkage. This induces major conformational changes in the backbone by comparison to the linear cellulosic β1→4 linkages. Indeed, the rheological properties of both acetan and xanthan have been hypothesized to be mainly guided by the cellulosic nature of the polysaccharide backbone responsible for the formation of helices in their tridimensionnal structures (Kirby, A.R et al. (1995) Microscopy. *Biophys. J.* **68,** 360-363). It is therefore likely that the properties measured for the polysaccharide are significantly different as a result of different backbone geometry.

In addition, the packing of two different sidechains can have a direct influence on the macroscopic rheological properties of the polysaccharide. Another important difference with acetan may be the absence of detectable acetylation.

Also, the presence of different sidechains may increase molecular interactions. It is also known that rheological properties are influenced by the length of the sidechains, for example. Indeed, it has been shown on variants of the acetan structure deficient in the sidechain sugar residues which are produced by mutants of *Gluconacetobacter* obtained by chemical mutagenesis, that decreasing the length of the sidechain enhances the solution viscosity (Ridout, M.J. et al., (1994) *Int. J*. *Biol. Macromol*., 16, 6, 324-330). The polysaccharide according to the present invention may also have a structure deficient in the sidechain sugar residues. Accordingly, the invention also relates to polysaccharide in which at least one sidechain is reduced from at least one residue.

Rheological properties of solutions of the polysaccharide were determined and compared to acetan and xanthan, currently one of the major texturizing agent used in the food industry (see examples). The polysaccharide was shown to exhibit different properties that on the basis of the structure can most likely be attributed to the structure, the absence of acetylation and the non-cellulosic backbone.

The rheological behavior of the polysaccharide is only slightly less shear-resistant at low concentration than xanthan, but more shear-resistant at higher shears. The likely consequence concerning the postulated mechanism of gellation is that the polysaccharide makes a different type of intermolecular interactions than acetan and xanthan. The formation of helical structures and helix-coil transition described for acetan must differ. Moreover, it is important to note that the viscosity was almost not affected by NaCl concentrations in the range 0.01 - 1 M.

Considering the polysaccharide's structure together with its extremely high resistance to important stresses, it is likely that sidechain intertwinning are the basis of resistance and gellifying properties.

The potential of this polysaccharide as a texturizing food additive, resulting from its production by a food-grade bacterium and its favourable texturizing properties, were clearly demonstrated.

According to another aspect, an isolated and purified *Gluconacetobacter* strain that is characterised by a high production of polysaccharide which has a xanthan-like structure with a non-cellulosic backbone with 4-beta and 6-beta linkages, non or very little acetylation and having at least one sidechain per repeating unit, and a low or no production of cellulose, is concerned.

In fact, wild strains of *G*. *xylinus* have been shown to produce acetan alone, and also acetans of different structures, but it has been surprisingly found a bacterial polysaccharide, which has:
- a non-cellulosic backbone with 4-beta and 6-beta linkages
- non or very little acetylation
- the presence of at least one sidechain per repeating unit.

Preferably, the *Gluconacetobacter* strain produces a polysaccharide which has the above described traits.

In a most preferred embodiment, the *Gluconacetobacter* strain is an *Gluconacetobacter spp.* strain which has been deposited on August 6, 1999 under the number CNCM I-2281 at the Institut Pasteur, 28 rue du Docteur Roux, F-75024 Paris cédex 15, FRANCE).

Said *Gluconacetobacter* strain is isolated from apple wine. The *G. xylinus* CNCM I-2281 strain produces under normal conditions, insoluble cellulose and a high-molecular-mass, highly soluble and texturizing heteropolysaccharide composed of glucose (Glc), rhamnose (Rha), mannose (Man) and glucuronic acid (GlcA) in the molar ratio of 7.3:1.4:1:1. Growth temperature conditions are between 15 and 34 °C, preferably a growth temperature of 28 °C. A pH comprised between 3.0 and 7.0, preferably about 4.0, applied during a fermentation time of 3-4 days under vigorous agitation, allows thereafter the production of the polysaccharide and no cellulose production (no cellulose was detected during fermentation). The structure of the polysaccharide produced by CNCM I-2281, was determined by chemical analysis, mass spectrometry and nuclear magnetic resonance spectroscopy. The repeating unit of the polysaccharide produced by this strain is presented figure 1.

The high production of polysaccharide and low or no production of cellulose by the *Gluconacetobacter sp.* strain allows its application in various fields, especially the preparation of food or pet food products or its incorporation in food or pet food products. Moreover, contrary the non-food grade plant pathogen *Xanthomaonas campestris* which produces xanthan, *Gluconacetobacter* are not pathogenic, and no reports were found suggesting induction of any allergic responses.

According to a further aspect, the invention relates to the use of such polysaccharide and/or said strain in various food or pet food products or for the preparation of various food or pet food products.

Preferably, the polysaccharide according to the invention may be used for the preparation of self-thickened vinegar and has various applications for the preparation of salad dressings, sauces, ketchup, mustard, etc.

Said polysaccharide can also be used for the preparation of fermented fruits or vegetables juices such as fermented "self-textured" tomato juice that can be used for ketchup with a dietary fibre content and improved texture, milk drinks supplemented with extracellular polysaccharide rich, fermented foods and vegetables (papes/compotes, juices, food preparations for ice-cream, etc.), as a polysaccharide-containing powder obtained by spray-drying, that find applications as a thickener in dehydrated products (such as soups and sauces).

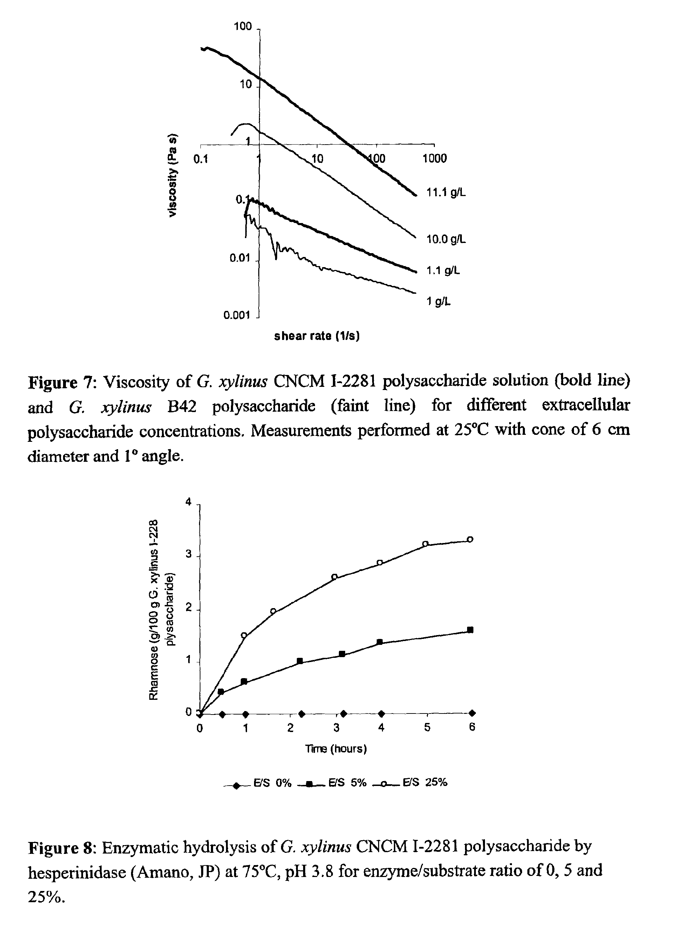

Also, the polysaccharide according to the invention may be used as an intermediate product for the isolation of rhamnose that allows increased levels of flavour in various food products. At acidic pH and high temperature, rhamnose is a precursor of furaneol and/or thiofuraneol. In order to release rhamnose, the isolated extracellular polysaccharide may be hydrolysed by enzymatic reaction or by acidic hydrolysis in very mild conditions, preferably at a pH of about 2-4 and moderate heating of about 90°C, for example, in a medium containing amino-acids proteins and/or polypeptides, during a time sufficient for at least liberating one rhamnose residue per repeating unit which will generate furaneol and/or thiofuraneol. Figure 8 shows the release of free rhamnose during enzymatic hydrolysis with hesperinidase (Amano, JP) at 75°C, pH 3.8 for enzyme/substrate ratio of 0, 5 and 25%.

The polysaccharide according to the present invention may be present in the food or pet food product in an amount of from about 0.01% to about 5%, and more preferably from 0.1% to 2%.

The strain according to the invention may be used in the food or pet food product in an amount of at least 1.10⁶ cfu/ g and more preferably from 10⁷ to 10⁸ cfu/ g.

Also, the invention relates to a food or pet food product or composition comprising at least the polysaccharide as described above and/or the bacterial strain as described above.

According to a last aspect, the present invention provides a fermentation process that permits advantageously the control of optimum biomass concentration and polysaccharide concentration by the *Gluconacetobacter* spp. strain according to the invention, wherein said *Gluconacetobacter* spp. strain is maintained under agitation in a growth medium containing salts and two different substrates S1 and S2 as carbon sources, S1 being a source of carbon for the production of biomass and S2 being a source of carbon for the production of polysaccharide.

According to a preferred embodiment, the substrate S1 used as a source of carbon for the production of the biomass is selected from the group consisting of ethanol, acetate, glycerol, succinic acid, citric acid and any organic acid containing 2 or 3 carbon atoms and intermediates of glycolysis and Tri Carboxylic Acid cycle (Krebs cycle), and any mixture of these substrates (referred as S1).

According to another preferred embodiment, the substrate S2 used as a source of carbon for the production of acetan is selected from the group consisting of glucose, fructose, saccharose, or any other sugar or combination of sugars (referred as S2).

Advantageously, in the fermentation process according to the invention and in order to obtain high biomass and polysaccharide concentrations, a high concentration of the substrates S1 and S2 can be present in the growth medium, and in an excess of dissolved oxygen.

### Isolation of the polysaccharide according to the invention

After growth on defined medium (Peters, H.U. et al., (1989) Biotechnol. Bioeng, 34, 1393-1397) with sucrose and ethanol, culture medium was harvested after sucrose consumption and centrifuged (30 min, 5000 rpm, 4°C) to remove cells. To precipitate soluble proteins, 250 g of trifluoroacetic acid was added per liter of supernatant, stirred for 1 h at 4°C. After centrifugation (30 min, 5000 rpm, 4°C) pH was adjusted to neutrality with NaOH pellets and 2 volumes of ice cold ethanol were added. After stirring and cooling to 4°C, precipitated extracellular polysaccharide was recovered by centrifugation (30 min, 5000 rpm, 4°C). Precipitate was dissolved in distilled water and dialysed against demineralized water for two days (molecular weight cut-off 6000-8000 Da) and lyophylized.

### Isolation of the strain according to the invention

The following media were described in the literature for selective and non-selective isolation of extracellular polysaccharide producing *Gluconacetobacter* strains: RAE (Sokollek, S.J. and Hammes, W.P. (1997) Appl. Microbiol. 20), SH (Hestrin, S., and Schramm, M., (1954) Biochemical journal 58: 345-352), and AJYE (Passmore, S.M., and Carr, J.G., (1974) J. Appl. Bacteriol. 38: 151-158) are best suited for isolation. These media were used to isolate strains of the genus *Gluconacetobacter* from running vinegar fermentations and from turbid vinegar. In static cultures the strains produced simultaneously high amounts of cellulose and polysaccharide. Studies with shaking cultures revealed that two of these isolates produced preferentially the polysaccharide according to the invention.

Under non-optimised fermentation conditions the culture achieved a total polysaccharide concentration > 8 g/l in the broth supplemented with about 1% acetic acid or ethanol.

Reproducible growth curves and reproducible polysaccharide production were obtained for several experiments. Collected from an agitated liquid culture, isolated strain produces large amounts of polysaccharide on agar plates on a medium containing ethanol, yeast extract and saccharose. Selected strain was applied in pilot experiments for establishing optimum extracellular polysaccharide production in food matrices.

The formation of polysaccaride depended on the composition of the nutrients. The production rate of polysaccharide was increased by addition of ethanol to the culture broth. A high glucose to yeast extract ratio stimulates extracellular polysaccharide biosynthesis and reduces cell growth.

Selection for increased polysaccharide producers was carried out by measuring extracellular polysaccharide in the culture medium by using the following non-limiting techniques:
i) centrifugation of the culture supernatant to remove bacterial cells,
ii) precipitation of extracellular polysaccharide by addition of ethanol or isopropylalcohol,
iii) separation of the precipitate by filtration followed by vacuum-drying.

Preferred growth temperature of the *Gluconacetobacter* spp. strain according to the present invention is between 15 and 34°C, preferably at a growth temperature of 28°C, pH is between 3.0 and 7.0, preferably about 4.0, during a fermentation time of 3-4 days under vigorous agitation, which allows the production of extracellular polysaccharide and no cellulose production (no cellulose was detected during fermentation).

### Fermentation conditions controlling the biomass and the extracellular polysaccharide production by the bacterial strain according to the invention

The growth medium contains salts and two different substrates S1 and S2 as carbon sources, S1 being a source of carbon for the production of biomass and S2 being a source of carbon for the production of extracellular polysaccharide.

The substrate S1 used as a source of carbon for the production of the biomass is selected from the group consisting of ethanol, acetate, glycerol, succinic acid, citric acid and any organic acid containing 2 or 3 carbon atoms and intermediates of glycolysis and Tri Carboxylic Acid cycle produced during the Krebs cycle, and any mixture of these substrates.

The substrate S2 used as a source of carbon for the production of extracellular polysaccharide is selected from the group consisting of glucose, fructose, saccharose, or any other sugar or combination of sugars.

The salts contained in the medium are those for example according to Peters et al. Underagitated conditions it was found that with this strain, as well as with other strains (e.g. DSM 2004, DSM 6315, DSM 46604, NRLL B42), most biomass formation occurred during consumption of S1, whereas extracellular polysaccharide was subsequently produced during consumption of sugar S2. Therefore, final biomass concentration was mainly determined by initial concentration of S1 and final extracellular polysaccharide concentration was mainly determined by the initial concentration of S2 and could reach values up to 50 g/L with no cellulose detected. High biomass and extracellular polysaccharide concentrations were obtained under excess of dissolved oxygen in the medium liquid. This was obtained by sufficient stirring and aeration rate of the media.

The following examples are given by way of illustration only and in no way should be construed as limiting the subject matter of the present application. All percentages are given by weight unless otherwise indicated. The examples are preceded by a brief description of the figures.

### Figures

**Figure 1** Structure of the polysaccharide gluconacetan produced by *G. xylinus* CNCM I-2281.
**Figure 2** Chemical structures of the bacterial polysaccharides (a) xanthan, (b) acetan and (c) CR1/4 (acetan variant secreted by *G. xylinus* strain CR1/4 (Ridout, M.J. et al., (1994) *Int. J. Biol. Macromol.* Vol 16, number 6).
**Figure 3 (top)** Structure of the gluconacetan polysaccharide produced by *G*. *xylinus* CNCM I-2281 with monosaccharide units identified by their residue letter code (**A** to **I**) **(bottom)** 1D ¹H NMR spectra of the polysaccharide produced by *G*. *xylinus* CNCM I-2281 recorded in ²H₂O at 600 MHz and 67°C. Anomeric (H-1) resonances are identified by the corresponding residue letter code.
**Figure 4 (left)** (C-6, H-6) region and **(right)** *N*-acetyl methyl region from the PEP-HSQC spectra of **(top)** the gluconacetan polysaccharide produced by *G*. *xylinus* CNCM I-2281 and of **(bottom)** the acetan polysaccharide produced by the *G. xylinus* B42 strain. Spectra were recorded in ²H₂O at 600 MHz and 67°C.
**Figure 5:** Viscosity of *G. xylinus* CNCM I-2281 gluconacetan polysaccharide solutions for different extracellular polysaccharide concentrations. Measurements performed at 25°C with cone of 6 cm diameter and 1° angle.
**Figure 6:** Viscosity of *G. xylinus* CNCM I-2281 polysaccharide solutions (bold line) and xanthan solutions (faint line) for different extracellular polysaccharide concentrations. Measurements were performed at 25°C with cone of 6 cm diameter and 1° angle.
**Figure 7:** Viscosity of *G. xylinus* CNCM I-2281 polysaccharide solution (bold line) and *G. xylinus* B42 polysaccharide (faint line) for different extracellular polysaccharide concentrations. Measurements performed at 25°C with cone of 6 cm diameter and 1° angle.
**Figure 8:** Enzymatic hydrolysis of *G. xylinus* CNCM I-2281 polysaccharide by hesperinidase (Amano, JP) at 75°C, pH 3.8 for enzyme/substrate ratio of 0, 5 and 25%.

### Examples

### Example 1: Chemical Analysis of the polysaccharide produced by G. xylinus CNCM I-2281

Quantitative monosaccharide analyse of the polysaccharide produced by *G. xylinus* CNCM I-2281 was performed by HPLC after acid hydrolysis (2 N trifluoroacetic acid, 100°C, samples taken after 2, 4, 6 and 8 h).

For the methylation analysis, the polysaccharide produced by *G. xylinus* CNCM I-2281 was reduced by carbodiimide-activated reduction then was permethylated using methyliodide (Carpita, N.C., Shea, E.M., (1989) in: *Analysis of carbohydrates by GLC and MS,* Eds.: Bierman, C.J.; McGinnis, G.D., CRC Press, Boca Raton; Ciucanu, I. et al., Rapid Method for the Permethylation of Carbohydrates. *Carbohydr. Res.* **131,** 209-217 (1984). The resulting partially methylated alditol acetates were analyzed by gas-liquid chromatography coupled to a mass spectrometer.

### NMR Spectroscopy

Samples were dissolved in 99.96 atom% ²H₂O (Euriso-Top). All experiments were recorded on a three-channel Bruker DRX 600 MHz spectrometer equipped with an actively shielded pulsed-field z-gradient inverse triple-resonance probe. Chemical shifts are given in ppm by reference to the α-anomeric signal of external [¹³C-1]-glucose (δ_{H-1} 5.15 for H-1 and δ_{C-1} 92.90 for C-1).

Phase-sensitive two-dimensional experiments were recorded using TPPI ( Marion, D. et al., (1983) *Biochem. Biophys. Res. Commun.* **113,** 967-974, TOCSY (Braunschweiler, L. et al., (1983) *J*. *Magn. Reson.* **53**, 521-528) with mixing times between 15 ms and 90 ms, NOESY (Jeener, J. et al., (1979) *J. Chem. Phys.* **11,** 4546-4553; Anil Kumar, Ernst, R.R. et al. (1980) *Biochem. Biophys. Res. Commun.* **95,** 1-6) with mixing times between 50 ms and 250 ms, gradient sensitivity-enhanced ¹H-¹³C heteronuclear single-quantum coherence (PEP-HSQC) (Kay, L.E., et al. (1992) *J. Am. Chem. Soc.* **114,** 10663-10665), and cross-correlated dipole-dipole (CCDD) experiment (Vincent, S.J.F. and Zwahlen, C. (2000) *J. Am. Chem. Soc.* **122,** 8307-8308) for determining glycosidic linkages with constant-time durations of 10 to 40 ms.

A magnitude mode gradient-filtered ¹H-¹³C HMBC (Bax, A. et al. (1986) *J*. *Am. Chem. Soc.* **108,** 2093-2094) was recorded with a *J*-evolution time of 50 ms. The following number of complex points were acquired (F₁, F₂): 512 x 4096 (TOCSY and NOESY), 256 x 2048 (HSQC) and 512 x 4096 (CCDD and HMBC), with averaging over 16 scans (TOCSY and NOESY), 128 scans (HSQC) or 256 scans (CCDD and HMBC). Spectral widths (ω₁, ω₂) of either 3600 Hz x 3600 Hz (TOCSY and NOESY) or 3020 Hz x 3600 Hz (HSQC, CCDD and HMBC) were used. A 90° shifted square sine-bell was used in all cases, with zero-filling once. All data were processed using Bruker XWINNMR 2.6 software.

### Results

Monosaccharide analysis of the polysaccharide produced by *G*. *xylinus* CNCM I-2281. Acid hydrolysis with 2N trifluoroacetic acid at 100°C showed that the amount of glucuronic acid, as determined from the amount of its main degradation product present, was roughly equivalent to the amount of mannose generated by the procedure (for one mole of mannose, 1.22 mole of glucuronic acid were found).

Methylation analysis of the gluconacetan polysaccharide produced by *G*. *xylinus* CNCM I-2281 (Table I) indicated the presence of glucose, rhamnose and mannose in the molar ratio of 5.5:1.4:1. Two different branching Glc*p* residues were found, whereas only terminal Rha was found, indicating a complicated branched repeating unit.

**Table I.**

| Chemical analysis data of the gluconacetan polysaccharide produced by *G. xylinus* CNCM I-2281. | | | | | | |
|---|---|---|---|---|---|---|
| Monosacc. | structure (NMR, m=2) | | MSA | | methylation | |
| | # | % | # | % | # | % |
| Rha-(1-> | 3 | 15.8 | 2.5 | 13.2 | 2.6 | 13.5 |
| ->2)-αMan-(1-> | 2 | 10.5 | 2.3 | 12.1 | 2.3 | 12.0 |
| ->4)-βGlcA-(1-> | 2 | 10.5 | 2.3 | 12.1 | 2.8 | 12.8 |
| | | | | | | |
| all Glc ^{a} | 12 | 63.2 | 11.9 ^{a} | 62.6 ^{a} | 11.7 | 61.7 |
| ->4)-βGlc-(1-> | 3 | 15.8 | - | - | 2.7 | 14.0 |
| ->6)-α-Glc-(1-> | 3 | 15.8 | - | - | 2.9 | 15.2 |
| ->6)-βGlc-(1-> | 3 | 15.8 | - | - | 2.9 | 15.2 |
| ->3,4)-βGlc-(1-> | 2 | 10.5 | - | - | 3.0 | 16.1 |
| ->3,6)-βGlc-(1-> | 1 | 5.3 | - | - | 0.2 | 1.2 |
| Total | 19 | 100 | 19 | 100 | 19 | 100 |

| | | | | | | |
|---|---|---|---|---|---|---|
| ^{a} The monosaccharide analysis only determines the total amount of any monosaccharide species, for example the total glucose content. | | | | | | |

### NMR Spectroscopy

The 1D ¹H NMR spectra of the polysaccharide produced by *G. xylinus* CNCM I-2281 (Figure 3) showed seven anomeric proton resonances with relative integrals 3:1.4:0.8:4.4:1.8:2.6:3.6 . The linewidths vary significantly between different anomeric resonances, from 5 Hz to 30 Hz, indicating widely variable dynamical motions for various monosaccharide units.

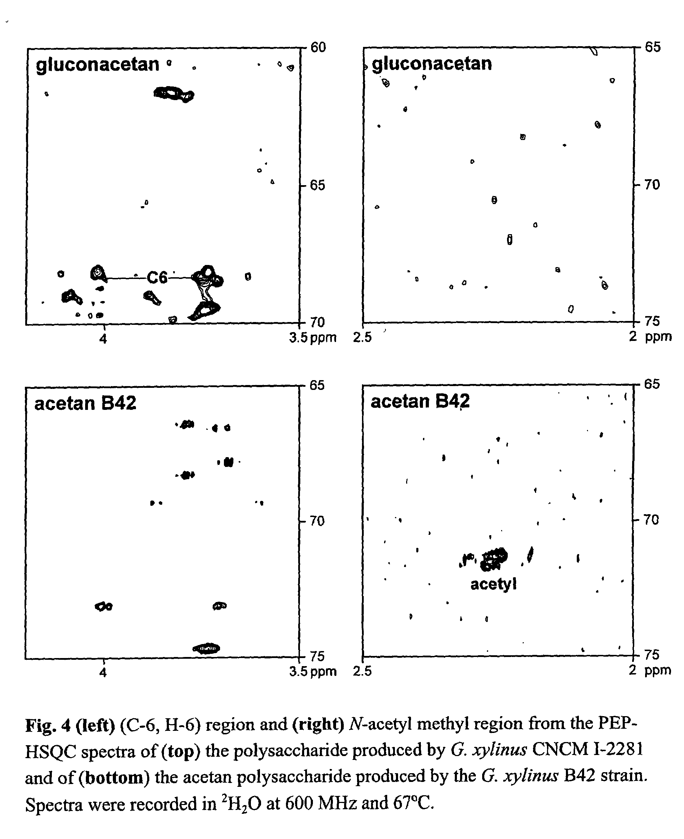

After observation of two-dimensional spectra, nine monosaccharide components within the repeating unit were identified and designated **A** to **I** following decreasing anomeric proton chemical shifts. Ring forms (hexose or pyranose) and anomeric configurations were deduced from H-1 chemical shifts and one-bond C-1, H-1 scalar couplings measured on the CCDD spectra. No *N*-acetyl methyl signal were observed around 2.08 ppm (Figure 4, top right) indicating the absence of acetylation. This was confirmed by the shifts of carbon positions which were acetylated in the polysaccharide acetan and which were shifted to non-subsituted position in the polysaccharide produced by *G. xylinus* CNCM I-2281 (cf. Table II). A set of standard polysaccharide NMR experiments were recorded on the polysaccharide produced by *G. xylinus* CNCM I-2281 at 67°C. The ¹H and ¹³C NMR assignments for the polysaccharide produced by *G*. *xylinus* CNCM I-2281 at 67°C are collected in Table II.

**Table II**

| ¹H and ¹³C NMR chemical shifts of the polysaccharide produced by *G. xylinus* CNCM I-2281 determined in ²H₂O at 67°C. The values are given in ppm relative to external [¹³C-1]glucose (δ_{H-1(α)} 5.15 and δ_{C-1(α)} 92.90).^{a} | | | | | | | | | |
|---|---|---|---|---|---|---|---|---|---|
| | | H-1 | H-2 | H-3 | H-4 | H-5 | H-6a | H-6b | CH₃ |
| | | C-1 | C-2 | C-3 | C-4 | C-5 | C-6 | | |
| **A** | →6)-α-D-Glc*p*-(1→ | 5.46 | 3.55 | 3.68 | 3.80 | 3.79 | **4.07** | **3.87** | |
| | | 99.8 | 72.6 | 73.8 | 71.4 | 72.1 | **69.0** | | |
| **B** | →2)-α-D-Man*p*-(1→ | 5.27 | **4.33** | 3.83 | 3.98 | 3.73 | 4.00 | 3.71 | |
| | | 100.8 | **79.6** | 70.7 | 73.8 ^{b} | 69.6 | 61.7 ^{c} | | |
| **C** | →3,6)-β-D-Glc*p*-(1→ | 5.23 | 3.36 | **4.38** | 3.53 | 3.64 | **3.99** | **3.73** | |
| | | 100.9 | 74.4 | **83.8** | 72.6 | 75.9 | **68.0** | | |
| **D** | α-L-Rha*p*-(1→ | 4.85 | 4.00 | 3.80 | 3.45 | 3.76 | | | 1.20 ^{d} |
| | | 101.7 | 71.1 | 71.4 | 73.3 | 69.6 | | | 17.7 ^{d} |
| **E** | α-L-Rha*p*-(1→ | 4.85 | 4.00 | 3.80 | 3.45 | 3.76 | | | 1.31 ^{d} |
| | | 101.7 | 71.1 | 71.4 | 73.3 | 69.6 | | | 17.7 ^{d} |
| **F** | →3,4)-β-D-Glc*p*-(1→ | 4.63 | 3.26 | 3.49 | **3.83** | 3.43 | 3.61 | 3.77 | |
| | | 97.2 | 75.1 | 76.8 | **80.6** | 76.9 | 61.8 | | |
| **G** | →3,4)-β-D-Glc*p*-(1→ | 4.58 | 3.41 | **3.81** | **3.87** | 3.66 | 4.02 | 3.83 | |
| | | 103.6 | 73.2 | **81.5** | **76.2** | 75.5 | 60.8^{c} | | |
| **H** | →4)-β-D-Glc*p*A-(1→ | 4.50 | 3.44 | 3.76 | **3.86** | 3.85 | | | |
| | | 103.3 | 73.8 | 76.8 | **81.2** | 76.6 | | | |
| **I** | →6)-β-D-Glc*p*-(1→ | 4.48 | 3.34 | 3.51 | 3.40 | 3.57 | **4.00** | **3.72** | |
| | | 103.7 | 74.1 | 76.8 | 70.9 | 75.9 | **68.1** | | |

| | | | | | | | | | |
|---|---|---|---|---|---|---|---|---|---|
| ^{a} Chemical shifts highlighted in bold typeface indicate positions at which a glycosidic link is identified based on differences to the corresponding reference chemical shifts. | | | | | | | | | |
| ^{b} Might indicate the presence of a substituent | | | | | | | | | |
| ^{c} In the acetan shifts, these positions were acetylated and shifted (**B**(C-6)=64.6p and **F**(C-6)=63.4p). | | | | | | | | | |
| ^{d} The peak intensity ratio in the PEP-HSQC was **D**(CH₃) : **E**(CH₃) 1.75:1 indicating that the long "acetan-like" sidechain **(D-I-A-H-B)** is twice more present than the shorter sidechain **(E-I-A).** | | | | | | | | | |

The ¹H assignment of the polysaccharide produced by *G. xylinus* CNCM I-2281 started from the anomeric (H-1) resonances of each residue **A** to **I** in the TOCSY spectra recorded with increasing mixing times (15 to 90 ms).

Connectivities from H-1 to H-2,3,4 were traced for all nine residues, but due to overlap of pairs of anomeric proton resonances (**D**(H-1) and **E**(H-1) at 4.85 ppm, **F**(H-1) and **G**(H-1) at 4.60 ppm, and **H**(H-1) and **I**(H-1) at 4.49 ppm) and their linewidths on the order of the chemical shifts difference (LW ~ 15 Hz = 0.02 ppm for Δδ ~0.02 ppm), a complete ¹H assignments were not obtained based on the TOCSY data alone.

Additional confirmations were obtained first from H-2,3,4,5 TOCSY traces, then from intra-monosaccharide intense NOESY cross-peaks and finally by assigning both the ¹H and the ¹³C resonances in the PEP-HSQC spectrum.

Resonances corresponding to aglyconic carbon atom involved in a glycosidic linkages were inferred from the ¹³C chemical shifts by identifying differences (> 5 ppm) in comparison to monosaccharide methyl glycoside references (Bock, K. et al. (1982) *Annu. Rep. NMR Spectrosc.* **13,** 1-57; Bock, K. et al. (1983) *Adv*. *Carbohydr. Chem. Biochem.* **41,** 27-66; Bock, K. et al. (1983) *Adv. Carbohydr*. *Chem. Biochem.* **42,** 193-225).

The sequence of the monosaccharide residues was deduced from the presence of cross-peaks in ¹H-¹³C CCDD, ¹H-¹³C HMBC spectra and NOESY spectra. Relevant cross-peaks are summarized in Table III.

**Table III**

| CCDD, HMBC and NOESY information available for the determination of interresidue correlations in the polysaccharide produced by *G*. *xylinus* CNCM I-2281.^{a} Cross-peaks identities are indicated by (ω₁, ω₂) atoms identifications.^{b} | | | | |
|---|---|---|---|---|
| CCDD & HMBC | | OESY | | Linkages |
| **A**(H-1) | **H**(C-4) | **A**(H-1) | **H**(H-2,3,4,5) | **A**-(1→4)-**H** |
| **A**(C-1) | **H**(H-4) | | | α-D-Glc*p*-(1→4)-β-D-Glc*p*A |
| **A**(H-1) | **C**(C-3) | **A**(H-1) | **C**(H-2,4) | **A**-(1→3)-**C** |
| **A**(C-1) | **C**(H-3) | **C**(H-3) | **A**(H-6b) | α-D-Glc*p*-(1→3)-β-D-Glc*p* |
| **B**(H-1) | **G**(C-3) | **B**(H-1) | **G**(H-1,2,3,4,5) | **B**-(1→3)-**G** |
| **B**(C-1) | **G**(H-3) | | | α-D-Man*p*-(1→3)-β-D-Glc*p* |
| | | **C**(H-1) | **F**(H-3) | **C**-(1→4)-**F** |
| | | **C**(H-3) | **F**(H-4) | β-D-Glc*p*-(1→4)-β-D-Glc*p* |
| **D**(H-1) | **I**(C-6) | **D**(H-1) | **I**(H-2,4,6a,6b) | **D**-(1→6)-**I** |
| **D**(C-1) | **I**(H-6a,6b) | | | α-L-Rha-(1→6)-β-D-G1c*p* |
| **E**(H-1) | **I**(C-6) | **E**(H-1) | **I**(H-2,4,6a,6b) | **E**-(1→6)-**I** |
| **E**(C-1) | **I**(H-6a,6b) | | | α-L-Rha-(1→6)-β-D-G1c*p* |
| **F**(H-1) | **C**(C-6) | **F**(H-1) | **C**(H-4,5,6a) | **F**-(1→6)-**C** |
| | | | | β-D-Glc*p*-(1→6)-β-D-Glc*p* |
| | | **F**(H-1) | **G**(H-4) | **F**-(1→4)-**G** |
| | | | | β-D-Glc*p*-(1→4)-β-D-Glc*p* |
| **G**(C-1) | **F**(H-4) | **G**(H-1) | **F**(H-4) | **G**-(1→4)-**F** |
| | | | | β-D-Glc*p*-(1→4)-β-D-Glc*p* |
| **H**(H-1) | **B**(C-2) | **H**(H-1) | **B**(H-2) | **H**-(1→2)-**B** |
| **H**(C-1) | **B**(H-2) | | | β-D-Glc*p*A-(1→2)-α-D-Man*p* |
| **I**(H-1) | **A**(C-6) | **I**(H-1) | **A**(H-3,6a,6b) | **I**-(1→6)-**A** |
| **I**(C-1) | **A**(H-6a) | | | β-D-Glc*p*-(1→6)-α-D-Glc*p* |

| | | | | |
|---|---|---|---|---|
| ^{a} CCDD and HMBC provide the same information about glycosidic linkages (Vincent, S.J.F. and Zwahlen, C. (2000) *J. Am. Chem. Soc.* 122, 8307-8308), but using a different transfer mechanism: CCDD is based on cross-correlated dipole-dipole relaxation, while HMBC uses on the heteronuclear long-range scalar coupling accross the glycosidic linkages. | | | | |
| ^{b} ω₁ refers to the first (indirect) frequency dimension, while ω₂ refers to the second (direct) frequency dimension. | | | | |

In all cases, glycosidic linkages were assigned relying simultaneously on the methylation analysis data, the ¹³C NMR assignments and the connectivities. The most critical connections between monosaccharide units were first the "cellulosic" (β-D-Glc*p*-(1→4)-β-D-Glc*p*) linkages **C**-(1→4)-**F**, **F**-(1→4)-**G** and **G**-(1→4)-**F**, then the "non-cellulosic" (β-D-Glc*p*-(1→6)-β-D-Glc*p*) backbone linkage **F**-(1→6)-**C** and finally the two linkages from residue **A,** first to the sidechain glucuronic acid **H** (α-D-Glc*p*-(1→4)-β-D-Glc*p*A) and, second, to the backbone branching **C** residue (α-D-Glcp-(1→3)-β-D-Glc*p*). The three cellulosic backbone β14 linkages were the hardest to identify as a direct result from the broad peaks associated with residues **C**, **F** and **G**. The non-cellulosic backbone β16 linkage was clearly demonstrated by the presence of CCDD, HMBC and NOESY cross-peaks between the anomeric proton of **F** and the atoms at or near the aglyconic position 6 of **C** (see Table III), in addition to the chemical shifts of residue **C.** The isolation of the carbon **C**(C-6) chemical shift leaves no doubt concerning this connection, even though these peaks are weak (see Figure 4, top left). The two different linkages from residue **A** are demonstrated by several NOESYs and symmetry-related CCDD and HMBC cross-peaks (Table III).

In conclusion, based on chemical analysis and NMR spectroscopy, the structure of the repeating unit of the polysaccharide secreted by *G*. *xylinus* CNCM I-2281 can be formulated as represented in figure 3 (top). The structure can accomodate a varying ratio of cellulosic to non-cellulosic backbone linkages, i.e. different value for m in the structure of figure 1. Based on the chemical analysis, the ratio of β16 to β14 bond should be small (Table I). According to rhamnose methyle NMR peak intensities, this ratio should be high. The best match was for m=2 where three sidechains per repeating unit are present, two identical to the acetan sidechain **(D-I-A-H-B),** and one smaller sidechain **(E-I-A).**

The polysaccharide repeating unit presents three sidechains. Two are five monosaccharide long and are identical in their composition to the acetan sidechain. In the polysaccharide produced by *G. xylinus* CNCM I-2281, they are attached to a backbone β-D-Glc*p* branching residue through a α13 linkage. Contrarily to both acetan and the structurally-related xanthan polysaccharide, the polysaccharide produced by *G. xylinus* CNCM I-2281 additionally presents a different type of sidechain composed of three monosaccharide units also attached via a α1→3 linkage to a backbone β-D-Glc*p* branching residue. The identity of this additional sidechain is identical to the three terminal residues of the acetan sidechain. In the backbone, the β-D-Glc*p* were shown to be alternatively branched. Moreover, and in contrast to both acetan and xanthan, one backbone residue was found to have a β16 linkage. This induces major conformational changes in the backbone by comparison to the linear cellulosic β14 linkages. Indeed, the rheological properties of both acetan and xanthan have been hypothesized to be mainly guided by the cellulosic nature of the polysaccharide backbone responsible for the formation of helices in their tridimensionnal structures (Kirby, A.R et al. (1995) Microscopy. *Biophys. J.* **68,** 360-363). It is therefore likely that the properties measured for the polysaccharide produced by *G. xylinus* CNCM I-2281 are significantly different as a result of different backbone geometry. In addition, the close packing of two different sidechains are shown to have a direct influence on the macroscopic rheological properties of the polysaccharide. Another important difference with acetan is the absence of detectable acetylation.

### Example 2 : Rheology of the polysaccharide

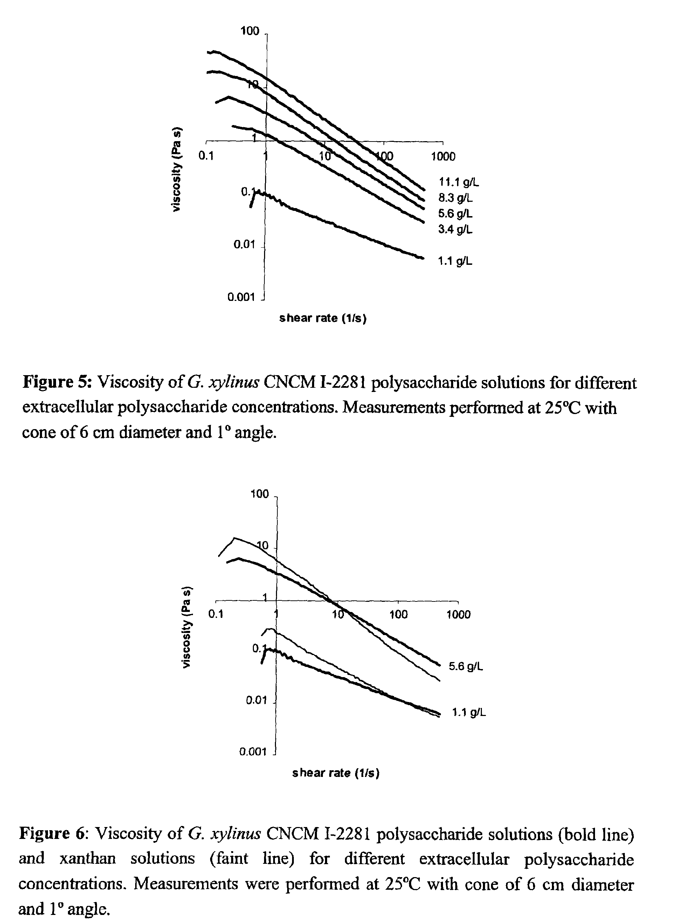

Purified extracellular polysaccharide samples were carefully dissolved in demineralized water. Xanthan (Rhodigel) was obtained from Meyhall (CH). Viscosity was measured at 25°C with a viscometer (SCL2 Carri Med rheometer, TA Instruments, New Castle, USA) equipped with a cone of 6 cm diameter and 1° angle. Shear rate was varied from 0.5 to 500 1/s. Figure 5 shows the profile of viscosity as a function of shear rate for the polysaccharide produced by *G. xylinus* CNCM I-2281.

The polysaccharide exhibits a shear thinning behaviour: at low shear rate the viscosity is high and almost constant; at high shear rate viscosity decreases continuously and reversibly (thixotropy). The viscosity was almost not affected by NaCl concentrations in the range 0.01 - 1 M.

Compared to xanthan solutions, the viscosity of the new polysaccharide is higher at high shear rate (Figure 6). For all shear rates tested, the viscosity of the new polysaccharide was significantly higher than the viscosity of the polysaccharide isolated from culture of *G*. *xylinus* B42 (Figure 7).

### Example 3: Use of extracellular polysaccharide according to the invention as stabiliser for particles or emulsions in food

Roughly 0.1% extracellular polysaccharide is used to suspend particles or droplets.

| | | |
|---|---|---|
| Food (e.g. salad) Dressing | vinegar, | 94.9% |
| | solids, | 5% |
| | extracellular polysaccharide (as in example 1). | 0.1% |

In a milk substitute 0.05% extracellular polysaccharide may be used to stabilise dispersions of dried whey or heat-processed soya protein in water.

### Example 4 : Ice cream composition using extracellular polysaccharide according the invention

| | |
|---|---|
| Fat | 10% |
| Serum solids | 11.7% |
| Sucrose | 11.0% |
| Corn syrup solids | 4.8% |
| extracellular polysaccharide (as in example 1) | 0.5% |
| Water | 62.0% |

### Example 5 : Preparation of fermented tomato paste with the strain of the invention

100 g of commercially available tomato paste (type Pummaro, ex STAR, Milan, Italy) are aseptically transferred into a sterile 300 ml size glass bottle and inoculated with 0.5% of a washed cell suspension of the *Gluconacetobacter* spp. strain and to a starting cell concentration of 2 X 10⁶ cells per gram (determined as colony forming units). Before inoculation, the pH of the tomato 20 matrix is adjusted to a value of 4.0 +/- 0.1.

Subsequently, the inoculated tomato paste is fermented for 24 hours at 28°C during which time samples are taken for carrying out analysis. After the fermentation is completed, the matrix is pasteurised for 30 minutes at 80°C and cooled to room temperature.

The fermented tomato paste obtained by using the *Gluconacetobacter* spp. strain has excellent properties concerning serum development and excellent organoleptic properties.

### Example 6: Flavour reaction

The isolated extracellular polysaccharide of example 1 is hydrolysed by enzymatic reaction or by acidic hydrolysis in very mild conditions, i.e. pH about 2-4 and moderate heating of about 90°C, in a medium containing amino-acids proteins and/or polypeptides, during a time sufficient for at least liberating one rhamnose residue per repeating unit which will generate furaneol and/or thiofuraneol.

### Example 7: Pet food product

A mixture is prepared from 73 % of poultry carcass, pig lungs and beef liver (ground), 16 % of wheat flour, 7 % of water, 2 % of dyes, flavours, vitamins, and inorganic salts. This mixture is emulsified at 12°C and extruded in the form of a pudding which is then cooked at a temperature of 90°C. It is cooled to 30°C and cut in chunks. 45 % of the chunks are mixed with 55 % of a sauce prepared from 98 % of water, 1 % of dye and 1 % of the extracellular polysaccharide gluconacetan. Tinplate cans are filled and sterilized at 125°C for 40 min.

## Claims

1. A bacterial extracellular polysaccharide, which has a xanthan-like structure with a non-cellulosic backbone with 4-beta and 6-beta linkages, non or very little acetylation and having at least one sidechain per repeating unit.

2. The polysaccharide according to claim 1, in which the non-cellulosic backbone has a repeating unit with the following structure: {[→4)-β-D-G1c-(1→4)-β-D-G1c-(1→]ₘ →6)-β-D-G1c-(1→4)-β-D-G1c-(1→ }n, in which *m* is an integer between 1 and 10, and *n* another integer defining the number of repeating units.

3. The polysaccharide according to claim 2, in which *m* is equal to 2.

4. The polysaccharide according to one of claims 1 to 3, wherein the sidechain is αL-Rha(1→6)-βD-G1c-(1→6)-αD-G1c-(1→4)- βD-G1cA-(1→2)- αD-Man, βD-G1c-(1→6)-αD-G1c-(1→4)-βD-G1cA-(1→2)-αD-Man, αD-Man, αD-G1c-(1→4)-βD-G1cA-(1→2)- αD-Man, αD-G1c-(1→4)- βD-G1cA, βD-G1cA-(1→2)- αD-Man, βD-G1c-(1→6)-αD-G1c-(1→4)- βD-G1cA, αL-Rha(1→6)-βD-G1c-(1→6)-αD-G1c-(1→4)- βD-G1cA, βD-G1cA, αL-Rha(1→6)-βD-G1c-(1→6)-αD-G1c, βD-G1c-(1→6)-αD-G1c, αD-G1c, αL-Rha(1→6)-βD-G1c, αL-Rha, or their derivatives.

5. The polysaccharide according to one of claims 1 to 4, in which the acetylation is less than 0.8 per site.

6. The polysaccharide according to one of claims 1 to 5, which has at least two different sidechains per repeatig unit, from which at least one is equivalent to the acetan sidechain or mutants of acetan sidechain and one different.

7. The polysaccharide according to one of claims 1 to 5, which has three different sidechains, from which two are equivalent to usual acetan and one different.

8. The polysaccharide according to claim 6, which has the structure presented in figure 1.

9. The polysaccharide according to claim 6 or 7, in which at least one sidechain is reduced from at least one residue.

10. The polysaccharide according to one of claims 1 to 9 obtainable by the strain *Gluconacetobacter* spp. having the deposit number CNCM I-2281.

11. An isolated and purified *Gluconacetobacter* spp. strain that is **characterised by** a high production of an polysaccharide which has a xanthan-like structure with a non-cellulosic backbone with 4-beta and 6-beta linkages, non or very little acetylation and having at least one sidechain per repeating unit, and a low or no production of cellulose.

12. The strain according to claim 11, wherein the polysaccharide has a non-cellulosic backbone having a repeating unit with the following structure: { [→4)-β-D-G1c-(1→4)-β-D-G1c-(1→]ₘ→6)-β-D-G1c-(1→4)-β-D-G1c-(1→ }n, in which *m* is an integer between 1 and 10, and *n* another integer defining the number of repeating units..

13. The strain according to claim 11 or 12, wherein the sidechain is the sidechain is αL-Rha(1→6)-βD-G1c-(1→6)-αD-G1c-(1→4)- βD-G1cA-(1→2)- αD-Man, βD-G1c-(1→6)-αD-G1c-(1→4)-βD-G1cA-(1→2)-αD-Man, αD-Man, αD-G1c-(1→4)-βD-G1cA-(1→2)- αD-Man, αD-G1c-(1→4)- βD-G1cA, βD-G1cA-(1→2)- αD-Man, βD-G1c-(1→6)-αD-G1c-(1→4)- βD-G1cA, αL-Rha(1→6)-βD-G1c-(1→6)-αD-G1c-(1→4)- βD-G1cA, βD-G1cA, αL-Rha(1→6)-βD-G1c-(1→6)-αD-G1c, βD-G1c-(1→6)-αD-G1c, αD-G1c, αL-Rha(1→6)-βD-G1c, αL-Rha, or their derivatives.

14. The strain according to one of claims 11 to 13, wherein the polysaccharide has the structure presented in figure 1.

15. The *Gluconacetobacter* spp. strain according to one of claims 11 to 14, having the deposit number CNCM I-2281.

16. The use of the polysaccharide according to one of claims 1 to 10 and/or the *Gluconacetobacter* strain according to one of claims 11 to 15 for the preparation of a food or pet food product.

17. The use according to claim 16, in which the amount of the polysaccharide is of about 0.01 to 5%.

18. The use according to claim 16, in which the amount of the *Gluconacetobacter* strain is of at least 1.10⁶ cfu/ g.

19. The use of the polysaccharide according to one of claims 1 to 10 as an intermediate product for the isolation of rhamnose intended for increasing levels of flavour in a food or pet food product.

20. Food or pet food product comprising at least the polysaccharide according to one of claims 1 to 10 and/or the *Gluconacetobacter* strain according to one of claims 11 to 15.

21. Food or pet food product according to claim 20, in which the amount of the polysaccharide is of about 0.01 to 5%.

22. Food or pet food product according to claim 20, in which the amount of the *Gluconacetobacter* strain is of at least 1.10⁶ cfu/ g.

23. A fermentation process of biomass and polysaccharide by the bacterial strain according to one of claims 5 to 7 **characterised in that** it comprises the step of submitting a culture of said strain to agitation in a growth medium containing salts and two different substrates (S1 and S2) as carbon sources, being respectively a source of carbon for the production of biomass (S1) and a source of carbon for the production of acetan (S2).

24. The fermentation process according to claim 23, **characterised in that** the substrate used as a source of carbon for the production of the biomass is selected from the group consisting of ethanol, acetate, glycerol, succinic acid, citric acid and any organic acid containing 2 or 3 carbon atoms and intermediates of glycolysis and Tri Carboxylic Acid cycle produced during Krebs cycle, and any mixture of these substrates.

25. The fermentation process according to claim 23 or 24, **characterised in that** the substrate used as a source of carbon for the production of acetan is selected from the group consisting of glucose, fructose, saccharose, or any other sugar or combination of sugars.
